# EUROPÄISCHE PATENTSCHRIFT

(11) **EP 2 950 875 B1**
(45) Veröffentlichungstag und Bekanntmachung des Hinweises auf die Patenterteilung: **20.05.2020**
(21) Anmeldenummer: 14700752.0
(22) Anmeldetag: 17.01.2014
(51) Int. Cl.: A61M 39/10, F16L 17/00, A61M 39/14, A61M 1/36

(54) **MEDIZINISCHE VORRICHTUNG MIT EINER BUCHSEN-EINHEIT ZUM ANSCHLUSS EINER STECKER-EINHEIT EINER VORRICHTUNG ZUR BEREITSTELLUNG VON MEDIZINISCHEN FLÜSSIGKEITEN**
MEDICAL DEVICE COMPRISING A SOCKET UNIT FOR CONNECTING A CONNECTOR UNIT OF A DEVICE FOR PROVIDING MEDICAL LIQUIDS
DISPOSITIF MÉDICAL AVEC UNE UNITÉ DOUILLE POUR CONNECTER UNE UNITÉ FICHE D'UN DISPOSITIF POUR METTRE À DISPOSITION DES LIQUIDES MÉDICAUX

(30) Priorität: 29.01.2013 DE 102013001438; 29.01.2013 US 201361757786 P
(43) Veröffentlichungstag der Anmeldung: 09.12.2015
(73) Patentinhaber: Fresenius Medical Care Deutschland GmbH, 61352 Bad Homburg (DE)
(72) Erfinder: MARTERSTOCK, Stefan Konrad, 97337 Dettelbach (DE)
(74) Vertreter: Oppermann, Frank
(86) Internationale Anmeldenummer: PCT/EP2014/050948
(87) Internationale Veröffentlichungsnummer: WO 2014/118023

(56) Entgegenhaltungen:
- EP-A2- 0 575 970
- EP-A2- 0 575 970
- US-A1- 2007 066 940
- US-A1- 2007 179 473
- US-A1- 2007 179 473
- US-A1- 2010 270 792

## Beschreibung

Die Erfindung betrifft eine medizinische Vorrichtung mit einer Buchsen-Einheit zum Anschluss einer Stecker-Einheit einer Vorrichtung zur Bereitstellung von medizinischen Flüssigkeiten, wobei die medizinische Vorrichtung insbesondere eine extrakorporale Blutbehandlungsvorrichtung, beispielsweise extrakorporale Dialysevorrichtung oder Vorrichtung zur Peritonealdialyse, und die medizinische Flüssigkeit insbesondere eine Dialysierflüssigkeit ist.

Zum Anschluss von externen Komponenten an medizintechnische Einrichtungen sind unterschiedliche Konnektoren bekannt. Der Zugang zu den medizintechnischen Einrichtungen erfolgt im Allgemeinen mittels Steckern, die in passende Buchsen der medizintechnischen Einrichtungen eingesteckt werden. Insofern verfügen die medizintechnischen Einrichtungen, die nachfolgend allgemein als medizinische Vorrichtungen bezeichnet werden, über eine entsprechende Buchsen-Einheit, während die externen Komponenten eine Stecker-Einheit aufweisen.

Zur Behandlung von nierenkranken Patienten finden Blutbehandlungsvorrichtungen Verwendung, zu denen insbesondere die bekannten extrakorporalen Dialysevorrichtungen oder Vorrichtungen zur Peritonealdialyse gehören. Zur Blutreinigung des Patienten ist die Bereitstellung von medizinischen Behandlungsflüssigkeiten erforderlich, die in Flüssigkeitsreservoirs bereitgestellt werden können, die an die Blutbehandlungsvorrichtungen angeschlossen werden. Der Anschluss des Flüssigkeitsreservoirs an die Blutbehandlungsvorrichtungen erfolgt mit einer Stecker-Einheit, die in eine Buchsen-Einheit der Blutbehandlungsvorrichtung eingesteckt wird.

Eine Vorrichtung zur Bereitstellung einer Behandlungsflüssigkeit ist aus der EP 0 575 970 A2 bekannt. Die bekannte Vorrichtung zur Bereitstellung von Dialysierflüssigkeit umfasst einen Beutel zur Aufnahme der Flüssigkeit, an dem eine Schlauchleitung angeschlossen ist, die an ihrem freien Ende mit einem Stecker verbunden ist. Die Dialysevorrichtung verfügt über eine Buchse, in die der Stecker eingesteckt wird. Mit dem Stecker und der Buchse können zwei Strömungsverbindungen hergestellt werden, um frische Dialysierflüssigkeit aus dem Beutel in die Dialysevorrichtung und verbrauchte Dialysierflüssigkeit zurück in den Beutel leiten zu können.

Der Anschluss der Vorrichtung zur Bereitstellung von medizinischen Flüssigkeiten an die Blutbehandlungsvorrichtung soll für das medizinische Personal möglichst einfach und sicher sein. Zur Desinfektion sollte sich die Buchsen-Einheit mit einer Spülflüssigkeit spülen lassen.

Die Buchsen-Einheit der aus der EP 0 575 970 A2 bekannten Blutbehandlungsvorrichtung verfügt über eine Einrichtung zur Erkennung der Position der Stecker-Einheit in der Buchsen-Einheit, die einen Reedkontakt in der Buchsen-Einheit und einen Magneten in der Stecker-Einheit umfasst.

Eine Aufgabe der Erfindung ist, die Versorgung von medizinischen Vorrichtungen, insbesondere Blutbehandlungsvorrichtungen, beispielsweise extrakorporale Dialysevorrichtungen oder Vorrichtungen zur Peritonealdialyse, mit medizinischen Flüssigkeiten für das medizinische Personal zu vereinfachen und die Sicherheit der Behandlung zu erhöhen.

Der Erfindung liegt insbesondere die Aufgabe zugrunde, einen Betriebszustand der medizinischen Vorrichtung einfach und sicher zu erkennen, insbesondere eine einfache und sichere Lösung für die Positionsbestimmung eines beweglichen Bauteils der Buchsen-Einheit der medizinischen Vorrichtung anzugeben.

Die Lösung dieser Aufgabe erfolgt erfindungsgemäß mit den Merkmalen des der unabhängigen Ansprüche. Die Unteransprüche betreffen vorteilhafte Ausführungsformen der Erfindung.

Die erfindungsgemäße medizinische Vorrichtung verfügt über eine Buchsen-Einheit, während die Vorrichtung zur Bereitstellung von medizinischen Flüssigkeiten über eine Stecker-Einheit verfügt.

Die Buchsen-Einheit und die Stecker-Einheit zeichnen sich dadurch aus, dass sich mit beiden Einheiten eine Strömungsverbindung zwischen der medizinischen Vorrichtung einerseits und der Vorrichtung zur Bereitstellung von medizinischen Flüssigkeiten andererseits einfach und sicher herstellen lässt.

Zur Herstellung der Strömungsverbindung verfügt die Buchsen-Einheit über mindestens ein Anschlussstück, während die Stecker-Einheit über mindestens einen Konnektor verfügt, so dass sich eine flüssigkeitsdichte Verbindung herstellen lässt, wenn der Konnektor an das Anschlussstück angeschlossen wird. Unerheblich ist, wie Anschlussstück und Konnektor ausgebildet sind. Anschlussstück und Konnektor können ihrerseits Stecker bzw. Buchsen sein.

Die medizinische Vorrichtung sieht einen automatisierten Anschluss der Stecker-Einheit an die Buchsen-Einheit mit einer Anschluss-Einrichtung zur Herstellung einer Verbindung zwischen dem mindesten einen Anschlussstück und dem mindesten einen Konnektor vor.

Die Anschluss-Einrichtung umfasst eine Antriebs-Einheit, die mindestens einen beweglichen Antriebskörper aufweist, der eine translatorische und/oder rotatorische Bewegung ausführt, um die Konnektion von Buchsen-Einheit und Stecker-Einheit vorzunehmen.

Darüber hinaus verfügt die Anschluss-Einrichtung über eine Positionserfassungs-Einrichtung zur Erfassung der linearen Position oder der Winkelstellung des Antriebskörpers. Die Erkennung der Position oder Winkelstellung erlaubt die Überwachung des Betriebszustandes oder die Steuerung der medizinischen Vorrichtung.

Eine bevorzugte Ausführungsform der Erfindung sieht vor, dass die Antriebs-Einheit der Anschluss-Einrichtung über einen Antriebskörper verfügt, der Mittel zum lösbaren

Verbinden der Stecker-Einheit mit der Buchsen-Einheit aufweist. Die Mittel zum lösbaren Verbinden der Stecker-Einheit mit der Buchsen-Einheit weisen vorzugsweise ein Aufnahmestück zum Einstecken eines Ansatzstückes der Stecker-Einheit auf.

Eine weitere bevorzugte Ausführungsform sieht vor, dass das mindestens eine Anschlussstück der Buchsen-Einheit unter Bildung einer Spülkammer von einem Anschlussteil konzentrisch umschlossen ist, wobei die Buchsen-Einheit einen Verschlusskörper mit mindestens einem Verschlussstück zum Verschluss der mindestens einen Spülkammer aufweist.

Bei dieser Ausführungsform ist der Verschlusskörper an einem um eine Drehachse drehbaren Antriebskörper vorgesehen. Das Verschlussstück ist an dem Verschlusskörper im Abstand zu der Drehachse angeordnet, wobei der Verschlusskörper zwischen einer ersten und einer zweiten Drehstellung verdreht werden kann.

In der ersten Drehstellung liegen das mindestens eine Verschlussstück und der mindestens eine Anschlussteil bzw. das Anschlussstück auf einer gemeinsamen Achse, so dass durch eine Relativbewegung von Stecker-Einheit und Buchsen-Einheit eine Verbindung zwischen Verschlussstück und Anschlussteil zum Verschluss der Spülkammer herstellbar ist. In diesem Zusammenhang wird unter einer Relativbewegung von Verschlussstück und Anschlussteil eine Bewegung eines verschiebbaren Verschlussstücks auf einen feststehenden Anschlussteil oder eine Bewegung eines verschiebbaren Anschlussteils auf ein feststehendes Verschlussstück verstanden.

In der zweiten Drehstellung sind das mindestens eine Verschlussstück und der mindestens eine Anschlussteil bzw. das Anschlussstück zueinander versetzt angeordnet, so dass beim Einstecken der Stecker-Einheit in die Buchsen-Einheit eine Verbindung zwischen dem mindestens einen Konnektor der Stecker-Einheit und dem mindestens einen Anschlussstück der Buchsen-Einheit herstellbar ist.

In Abhängigkeit von der Winkelstellung des drehbaren Antriebskörpers können die für die Verbindung von Stecker-Einheit und Buchsen-Einheit oder die für die Spülung erforderlichen Aktionen gesteuert oder überwacht werden.

Für die Erfindung ist unerheblich, wie der Antriebkörper beschaffen ist. Der Antriebkörper kann jedes längsverschiebbare oder drehbare Bauteil der Anschluss-Einrichtung sein.

Die Positionserfassungs-Einrichtung für den Antriebskörper der Anschluss-Einrichtung zeichnet sich durch mindestens zwei im Abstand zueinander angeordnete ein Magnetfeld erzeugende Magnet-Elemente und mindestens ein das Magnetfeld der Magnet-Elemente erfassendes Sensor-Element aus, wobei die mindestens zwei Magnet-Elemente an dem Antriebskörper und das mindestens eine Sensor-Element ortsfest oder die mindestens zwei Magnet-Elemente ortsfest und das mindestens eine Sensor-Element an dem Antriebskörper angeordnet sind. Dabei ist die Auswerteinheit derart konfiguriert, dass die lineare Position oder Winkelstellung des Antriebskörpers auf der Grundlage des von den mindestens zwei Magnet-Elementen erzeugten und von dem mindestens einen Sensor-Element erfassten Magnetfeldes bestimmt wird. Die beiden Sensor-Elemente erlauben eine eindeutige Erkennung der linearen Position oder Winkelstellung über einen großen Bewegungsspielraum des Antriebskörpers.

Bei einer Ausführungsform ist ein erstes Magnet-Element und ein zweites Magnet-Element, die im Abstand zueinander mit einander entgegengesetzter Polarität angeordnet sind, und ein erstes Sensor-Element und ein zweites Sensor-Element, die im Abstand zueinander angeordnet sind, vorgesehen, wobei die Auswerteinheit derart konfiguriert ist, dass auf der Grundlage des von dem ersten Sensor-Element erfassten Magnetfeldes zwei mögliche lineare Positionen oder Winkelstellungen des Antriebskörpers bestimmt werden und auf der Grundlage des von dem zweiten Sensor-Element erfassten Magnetfeldes bestimmt wird, ob die mit dem ersten Sensor-Element bestimmte lineare Position bzw. Winkelstellung die erste oder zweite lineare Position bzw. Winkelstellung der zwei möglichen Positionen bzw. Winkelstellungen ist. Die Unterscheidung zwischen den beiden möglichen linearen Positionen bzw. Winkelstellungen kann auf der Grundlage des Vorzeichens des von dem zweiten SensorElements erfassten Magnetfeldes erfolgen.

Eine alternative Ausführungsform sieht eine Begrenzung der translatorischen bzw. rotarischen Bewegung des Antriebskörpers durch ein oder mehrere Anschlagelemente auf einen Bewegungsspielraum vor, in dem eine eindeutige Bestimmung der Position und/oder Winkelstellung möglich ist.

Bei einer weiteren alternativen Ausführungsform umfasst die Positionserfassungs-Einrichtung ein erstes Magnet-Element und ein zweites Magnet-Element, die im Abstand zueinander mit einander entgegengesetzter Polarität angeordnet sind, wobei für eine eindeutige Positions- bzw. Winkelbestimmung aber nur ein Sensor-Element vorgesehen ist. Hierfür ist die Auswerteinheit derart konfiguriert, dass auf der Grundlage des von dem Sensor-Element erfassten Magnetfeldes zwei mögliche lineare Positionen oder Winkelstellungen des Antriebskörpers bestimmt werden, und dass die Auswerteinheit derart konfiguriert ist, dass der Gradient der Änderung des von dem Sensor-Element erfassten Magnetfeldes bei einer Änderung der linearen Position bzw. Winkelstellung des Antriebskörpers bestimmt wird. Auf der Grundlage des Vorzeichens des Gradienten der Änderung kann eindeutig zwischen den beiden möglichen Positionen bzw. Winkelstellungen unterschieden werden.

Die Auswerteinheit kann ein erstes Steuersignal erzeugen, wenn die Positionserfassungs-Einrichtung eine erste vorgeschobene Position des Antriebskörpers detektiert, in der der mindestens eine Konnektor und das mindestens eine Anschlussstück getrennt sind, und ein zweites Steuersignal erzeugen, wenn die Positionserfassungs-Einrichtung eine zweite zurückgezogene Stellung detektiert, wenn der mindestens eine Konnektor an das mindestens eine Anschlussstück angeschlossen ist. Die Positionserkennung-Einrichtung kann auch Steuersignale erzeugen, wenn eine erste Drehstellung und eine zweite Drehstellung des Antriebskörpers detektiert werden.

Das Magnet-Element ist vorzugsweise ein Permanentmagnet. Es kann aber auch ein Elektromagnet sein. Das Sensor-Element ist vorzugsweise ein Hall-Sensor oder ein magnetisch resistiver Sensor oder kann auch jeder andere Sensor zur Detektion eines Magnetfeldes sein.

Die medizinische Vorrichtung kann eine Blutbehandlungsvorrichtung, insbesondere eine extrakorporale Dialysevorrichtung oder eine Vorrichtung zur Peritionealdialyse sein.

Im Folgenden werden Ausführungsbeispiele der Erfindung unter Bezugnahme auf die Figuren näher erläutert.

Es zeigen:
- Fig. 1:: eine Vorrichtung zur Bereitstellung einer medizinischen Flüssigkeit, insbesondere Dialysierflüssigkeit, zusammen mit einer Blutbehandlungsvorrichtung und einer Vorrichtung zum Befüllen der Vorrichtung zur Bereitstellung von Dialysierflüssigkeit in stark vereinfachter schematischer Darstellung,
- Fig. 2: die Stecker-Einheit der Vorrichtung zur Bereitstellung von Dialysierflüssigkeit zusammen mit der Buchsen-Einheit der Blutbehandlungsvorrichtung in perspektivischer Darstellung,
- Fig. 3: die Buchsen-Einheit von Fig. 2 in perspektivischer Darstellung, wobei die Buchsen-Einheit für einen Spülvorgang vorbereitet ist,
- Fig. 4: die Stecker-Einheit und die Buchsen-Einheit von Fig. 2 in geschnittener Darstellung, wobei die Buchsen-Einheit zum Anschluss der Stecker-Einheit vorbereitet ist,
- Fig. 5: einen Schnitt durch die Stecker-Einheit und Buchsen-Einheit von Fig. 2, wobei die Stecker-Einheit in die Buchsen-Einheit lose eingesetzt ist, und
- Fig. 6: einen Schnitt durch die Stecker-Einheit und Buchsen-Einheit von Fig. 2, wobei die Stecker-Einheit an die Buchsen-Einheit angeschlossen ist, so dass die Strömungsverbindungen hergestellt sind,
- Fig. 7: die Buchsen-Einheit von Fig. 2 in geschnittener Darstellung, wobei die Buchsen-Einheit für einen Spülvorgang vorbereitet ist,
- Fig. 8: eine Darstellung des Magnetfeldes bei einer Anordnung mit zwei Magnet-Elementen und einem Sensor-Element zur Erfassung der linearen Position,
- Fig. 9: eine vereinfachte Darstellung einer ersten Ausführungsform der Positionserfassungs-Einrichtung mit zwei Magnet-Elementen und zwei Sensor-Elementen zur Erkennung der linearen Position eines translatorischen Antriebskörpers, wobei sich der Antriebskörper in einer ersten Position befindet,
- Fig. 10: die Positionserfassungs-Einrichtung von Fig. 9, wobei sich der Antriebskörper in einer zweiten Position befindet,
- Fig. 11: eine vereinfachte Darstellung einer Ausführungsform der Positionserfassungs-Einrichtung mit zwei Magnet-Elementen und einem Sensor-Element zur Erkennung der Winkelstellung eines rotatorischen Antriebskörpers, wobei sich der Antriebskörper in einer ersten Position befindet,
- Fig. 12: die Positionserfassungs-Einrichtung von Fig. 11, wobei sich der Antriebskörper in einer zweiten Position befindet.

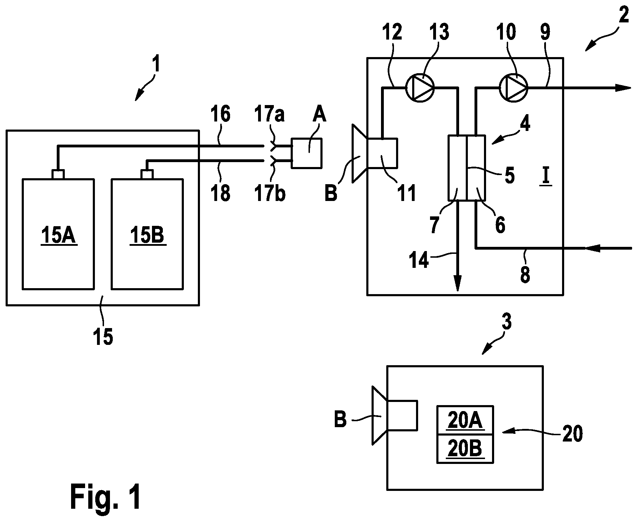

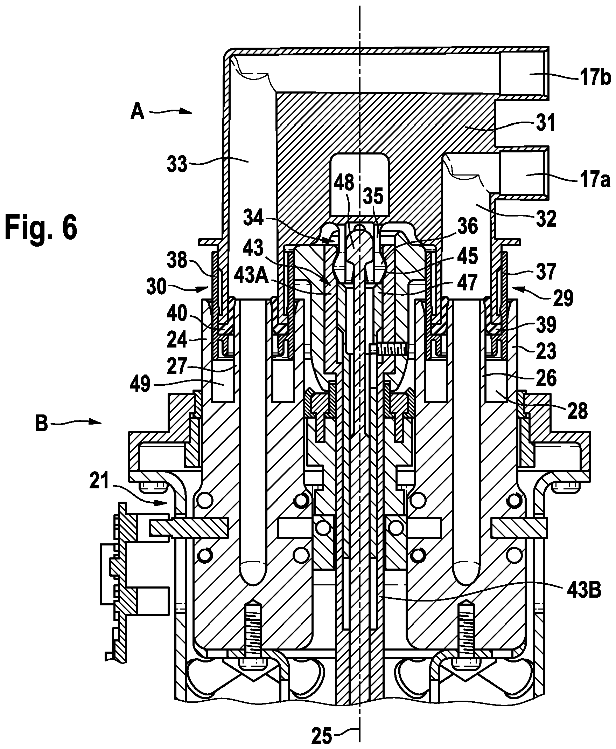

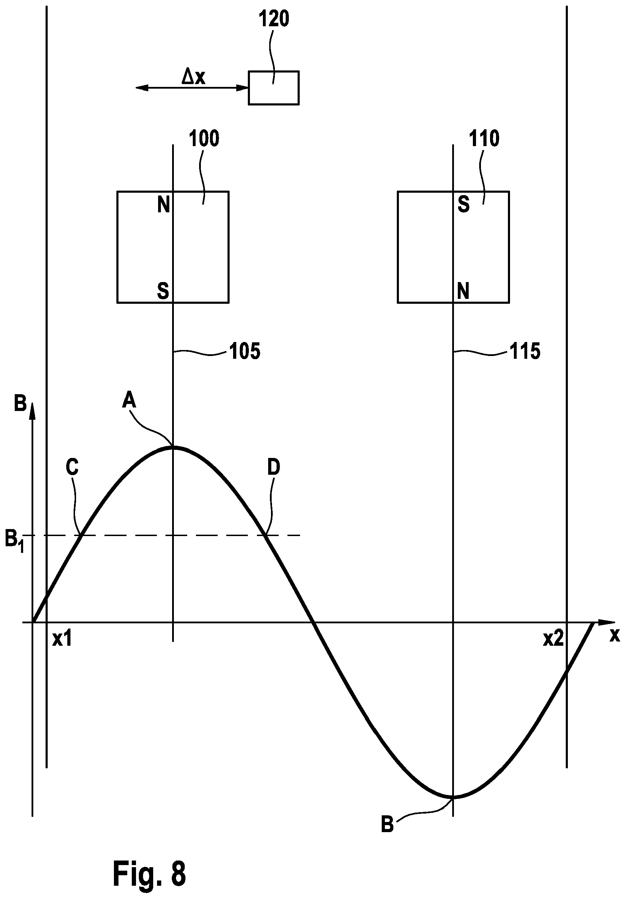

Nachfolgend wird zunächst die medizinische Vorrichtung mit der Buchsen-Einheit unter Bezugnahme auf die Figuren 1 bis 7 im Einzelnen beschrieben. Anschließend wird unter Bezugnahme auf die Figuren 8 bis 12 die Positionserfassungs-Einrichtung der Anschluss-Einrichtung der medizinischen Vorrichtung im Einzelnen beschrieben.

Fig. 1 zeigt in stark vereinfachter schematischer Darstellung eine Vorrichtung 1 zur Bereitstellung einer medizinischen Flüssigkeit, insbesondere Dialysierflüssigkeit, zusammen mit einer Blutbehandlungsvorrichtung 2 und einer Vorrichtung 3 zum Befüllen der Vorrichtung zur Bereitstellung von Dialysierflüssigkeit. Die Blutbehandlungsvorrichtung 2 als Beispiel für die erfindungsgemäße medizinische Vorrichtung kann eine extrakorporale Dialysevorrichtung oder eine Vorrichtung zur Peritonealdialyse sein. Bei dem vorliegenden Ausführungsbeispiel ist die Blutbehandlungsvorrichtung 2 eine Dialysevorrichtung, die über einen Dialysator 4 verfügt, der durch eine semipermeable Membran 5 in eine Blutkammer 6 und eine Dialysierflüssigkeitskammer 7 unterteilt ist. Von dem Patienten führt eine Blutzuführleitung 8 zu der Blutkammer 6 des Dialysators 4, während eine Blutrückführleitung 9, in die eine Blutpumpe 10 geschaltet ist, von der Blutkammer 6 zu dem Patienten führt. Die Blutzuführ- und -rückführleitung, 8, 9 bilden zusammen mit der Blutkammer 6 den extrakorporalen Blutkreislauf I der Dialysevorrichtung 2.

Die frische Dialysierflüssigkeit wird aus einem Dialysierflüssigkeitsreservoir 11 über eine Dialysierflüssigkeitszuführleitung 12, in die eine Dialysierflüssigkeitspumpe 13 geschaltet ist, zu der Dialysierflüssigkeitskammer 7 des Dialysators 4 geleitet, während verbrauchte Dialysierflüssigkeit über eine Dialysierflüssigkeitsabführleitung 14 aus der Dialysierflüssigkeitskammer abfließt.

Zur Bereitstellung frischer Dialysierflüssigkeit dient die Vorrichtung 1, die bei dem vorliegenden Ausführungsbeispiel zwei Beutel oder Kanister 15A und 15B aufweist. Beide Beutel oder Kanister 15A, 15B bilden eine Einheit 15, wobei der Beutel 15A vor der Dialysebehandlung mit frischer Dialysierflüssigkeit befüllt und der Beutel 15B leer ist.

Von dem Dialysierflüssigkeitsbeutel 15A führt eine Zulaufleitung 16 zu dem einen Anschluss 17a einer Stecker-Einheit A, während von dem anderen Anschluss 17b der Stecker-Einheit A eine Ablaufleitung 18 zu dem Leerbeutel 15B führt.

Die Stecker-Einheit A wird zur Bereitstellung von Dialysierflüssigkeit vor der Behandlung an eine Buchsen-Einheit B angeschlossen, die an der Blutbehandlungsvorrichtung 2 vorgesehen ist, so dass frische Dialysierflüssigkeit über die Zulaufleitung 16 dem Dialysierflüssigkeitsreservoir 10 zugeführt und verbrauchte Dialysierflüssigkeit über die Ablaufleitung 18 abgeführt werden kann. Die Dialysierflüssigkeit kann aber auch direkt der Dialysierflüssigkeitskammer 7 des Dialysators zugeführt werden.

Die Vorrichtung 1 zur Bereitstellung von Dialysierflüssigkeit wird an der Vorrichtung 3 mit frischer Dialysierflüssigkeit befüllt. Mit der Vorrichtung 3 zum Befüllen kann die Vorrichtung 2 zum Bereitstellen von Dialysierflüssigkeit auch entleert werden. Zur Aufnahme frischer Dialysierflüssigkeit dient ein Tank 20A und zur Aufnahme verbrauchter Dialysierflüssigkeit dient ein Tank 20B. Die erforderlichen Leitungen und Pumpen werden in der stark schematischen Darstellung nicht gezeigt.

Die Vorrichtung 3 zum Befüllen und Entleeren der Vorrichtung 1 zur Bereitstellung frischer und Aufnahme verbrauchter Dialysierflüssigkeit 1 verfügt über eine Buchsen-Einheit B, an der die Stecker-Einheit A der Vorrichtung 1 zur Bereitstellung von Dialysierflüssigkeit angeschlossen wird. Die Buchsen-Einheit B der Blutbehandlungsvorrichtung 2 und die Buchsen-Einheit B der Vorrichtung 3 zum Befüllen bzw. Entleeren können identisch oder unterschiedlich ausgebildet sein. Bei dem vorliegenden Ausführungsbeispiel sind die Buchsen-Einheiten B identisch ausgebildet. Beide Buchsen-Einheiten B sind derart ausgebildet, dass sich mit der Stecker-Einheit A der Vorrichtung 1 zur Bereitstellung von Dialysierflüssigkeit eine flüssigkeitsdichte Strömungsverbindung mit beiden Vorrichtungen 2 und 3 in beiden Richtungen für frische und verbrauchte Dialysierflüssigkeit herstellen lässt.

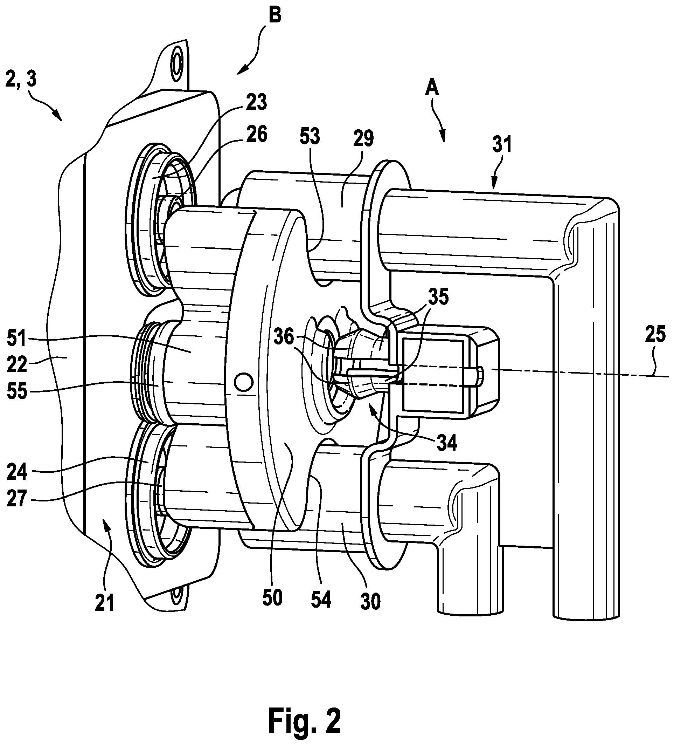

Nachfolgend wird die Stecker-Einheit A der Vorrichtung 1 zur Bereitstellung von Dialysierflüssigkeit zusammen mit der Buchsen-Einheit B unter Bezugnahme auf die Figuren 2 bis 7 im Einzelnen beschrieben.

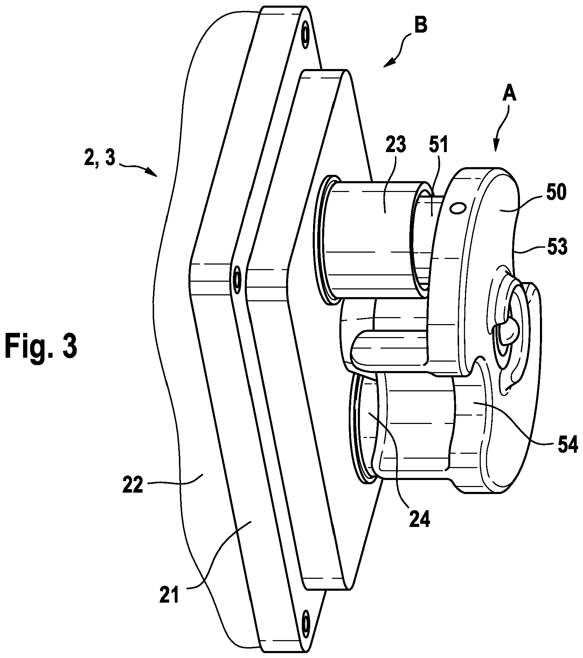

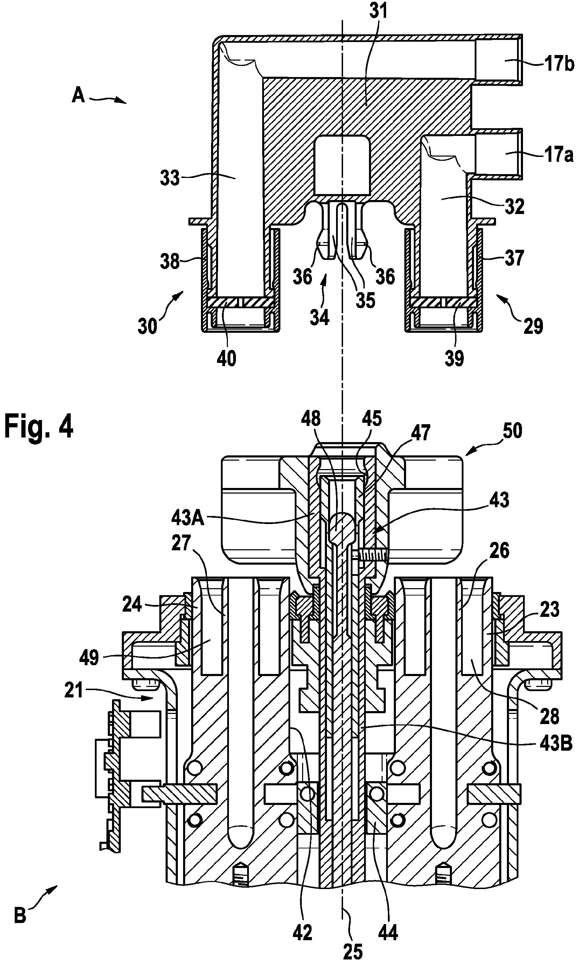

Die Figuren 2 und 3 zeigen die Stecker-Einheit A und die Buchsen-Einheit B in perspektivischer Darstellung, während die Figuren 4 bis 7 die Stecker- und Buchsen-Einheit A, B in geschnittener Darstellung zeigen.

Fig. 2 zeigt die Buchsen-Einheit B zusammen mit der Stecker-Einheit A in perspektivischer Darstellung. Mit der Stecker-Einheit A kann die Vorrichtung 1 zur Bereitstellung von Dialysierflüssigkeit einerseits an die Vorrichtung 3 zum Befüllen und Entleeren und andererseits an die Blutbehandlungsvorrichtung 2 angeschlossen werden.

Die Buchsen-Einheit B weist einen Gehäusekörper 21 auf, der in einer Gehäusewand 22 der Blutbehandlungsvorrichtung 2 oder der Vorrichtung 3 zum Befüllen eingesetzt ist. In dem Gehäusekörper 21 der Buchsen-Einheit B sind zwei zylindrische Anschlussteile 23, 24 vorgesehen, die in einer gemeinsamen Ebene zu beiden Seiten der zentralen Achse 25 der Buchsen-Einheit angeordnet sind. Die zylindrischen Anschlussteile 23, 24 umschließen jeweils konzentrisch ein Anschlussstück 26 bzw. 27, wobei das Anschlussstück 26 zum Zuführen frischer Dialysierflüssigkeit und das Anschlussstück 27 zum Abführen verbrauchter Dialysierflüssigkeit dient (Figuren 4 bis 7).

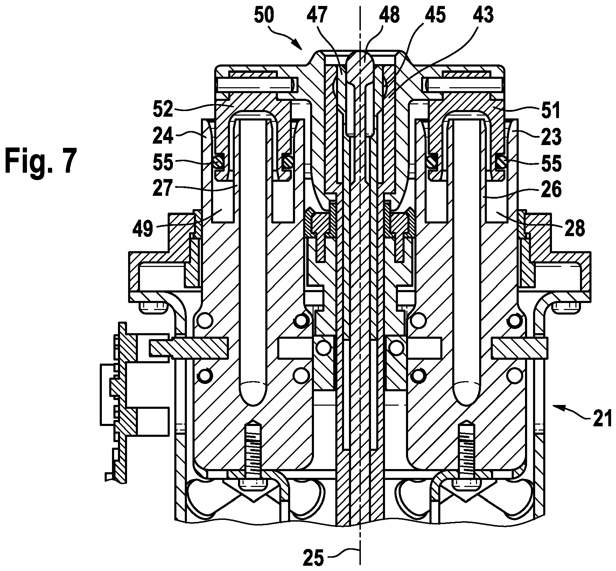

Von den beiden Anschlussteilen 23, 24 wird jeweils ein Raum umschlossen, der flüssigkeitsdicht verschlossen werden kann. Der flüssigkeitsdicht verschlossene Raum bildet eine Spülkammer 28, 49, durch die eine Spülflüssigkeit geleitet werden kann, die über nicht näher dargestellte Kanäle zuströmen oder abströmen kann (Fig. 7). Zum Spülen der Buchsen-Einheit B werden die Spülkammern 28, 49 von Spülflüssigkeit durchströmt. Dies wird nachfolgend noch im Einzelnen beschrieben.

Die Anschlussteile 23, 24 der Buchsen-Einheit B sind zusammen mit dem Anschlussstücken 27, 28 in dem Gehäusekörper 21 längsverschiebbar geführt, so dass die Anschlussteile und -stücke aus dem Gehäusekörper vorgeschoben bzw. in den Gehäusekörper zurückgezogen werden können. Die Antriebseinheit zum Vorschieben bzw. Zurückziehen der Anschlussteile und -stücke ist in den Figuren nicht im Einzelnen dargestellt. Sie kann eine elektromotorische oder hydraulische Antriebseinheit sein.

Die Stecker-Einheit A (Figuren 4 bis 6) der Vorrichtung 1 zur Bereitstellung frischer und zur Aufnahme verbrauchter Dialysierflüssigkeit verfügt über entsprechende Konnektoren 29, 30, die mit den Anschlussstücken 27, 28 flüssigkeitsdicht verbunden werden. Die Stecker-Einheit A weist einen Steckerkörper 31 auf, der die beiden Konnektoren 29, 30 verbindet. Der Steckerkörper 31 weist einen Zulaufkanal 32 auf, der an dem einen Konnektor 29 angeschlossen ist, und weist einen Ablaufkanal 33 auf, der an dem anderen Konnektor 30 angeschlossen ist. An dem Anschluss 17a des Zulaufkanals 32 ist die Zulaufleitung 16 und an dem Anschluss 17b des Ablaufkanals 33 ist die Ablaufleitung 18 der Vorrichtung 1 zur Bereitstellung von frischer bzw. Aufnahme verbrauchter Dialysierflüssigkeit angeschlossen. Zwischen den beiden Konnektoren 29, 30 befindet sich ein Ansatzstück 34, mit dem eine zunächst nur lose Verbindung zwischen der Stecker-Einheit A und der Buchsen-Einheit B hergestellt werden kann.

Das Ansatzstück 34 weist mehrere umfangsmäßig verteilt angeordnete Rastelemente 35 auf, die an einem Ende des Ansatzsstücks angeformt sind. An den Außenseiten der freien Enden der Rastelemente 35 sind Rastnasen 36 ausgebildet. Die Konnektoren 29 und 30 verfügen über Berührungsschutzhülsen 37 und 38, die auf die Konnektoren 29, 30 des Steckerkörpers 31 einrastend aufgesetzt sind. Die Konnektoren 29, 30 werden jeweils von einer Membran 39, 40 verschlossen, die von den Anschlussstücken 26, 27 der Buchsen-Einheit B durchdrungen wird.

Der Gehäusekörper 21 der Buchsen-Einheit B weist eine zentrale Ausnehmung 42 auf, in der ein rohrförmiges Aufnahmestück 43 angeordnet ist, in das sich das Ansatzstück 34 der Stecker-Einheit A einsetzen lässt. Das Aufnahmestück 43 ist mit einem Lager 44 um die Achse 25 drehbar gelagert, das in die zentrale Ausnehmung 42 des Gehäusekörpers 21 eingesetzt ist. Das Aufnahmestück 43 wird mit der Antriebseinheit gedreht.

Das rohrförmige Aufnahmestück 43 weist einen sich aus dem Gehäusekörper 21 erstreckenden vorderen Abschnitt 43A und einen sich in den Gehäusekörper erstreckenden hinteren Abschnitt 43B auf, wobei der vordere Abschnitt 43A einen größeren Außen- bzw. Innendurchmesser als der hintere Abschnitt 43B hat. An der Innenseite des vorderen Endes des vorderen Abschnitts 43A des Aufnahmestücks 43 sind umfangsgemäß verteilt angeordneten Ausnehmungen 45 vorgesehen, in die die Rastnasen 36 der Rastelemente 35 des Ansatzstücks 34 einrasten, wenn die Stecker-Einheit A lose auf die Buchsen-Einheit B aufgesetzt wird.

In dem rohrförmigen Aufnahmestück 43 ist längsverschiebbar ein als rohrförmiger Körper ausgebildetes Tastelement 47 geführt, dass mit einer nicht dargestellten Feder vorgespannt ist, so dass beim Einsetzen des Ansatzstücks 34 in das Aufnahmestück 43 das Tastelement 47 entgegen der Federspannung zurückgeschoben wird.

In dem rohrförmigen Tastelement 47 ist ein stiftförmiger Körper 48 zum Verriegeln des Ansatzstücks 34 in dem Aufnahmestück 43 geführt. Der stiftförmige Körper 48 kann mit einer nicht dargestellten Antriebseinheit in Längsrichtung vorgeschoben und wieder zurückgezogen werden, um das Ansatzstück 34 in dem Aufnahmestück 43 freizugeben bzw. zu verriegeln.

Fig. 4 zeigt die Buchsen-Einheit B in der Position, in der die Stecker-Einheit A lose auf die Buchsen-Einheit B aufgesetzt werden kann. Der stiftförmige Körper 48 ist in dem Aufnahmestück 43 zurückgezogen, so dass die Rastelemente 35 mit den Rastnasen 36 des Ansatzstücks 34 in das Aufnahmestück 43 mit den Ausnehmungen 45 einrasten können.

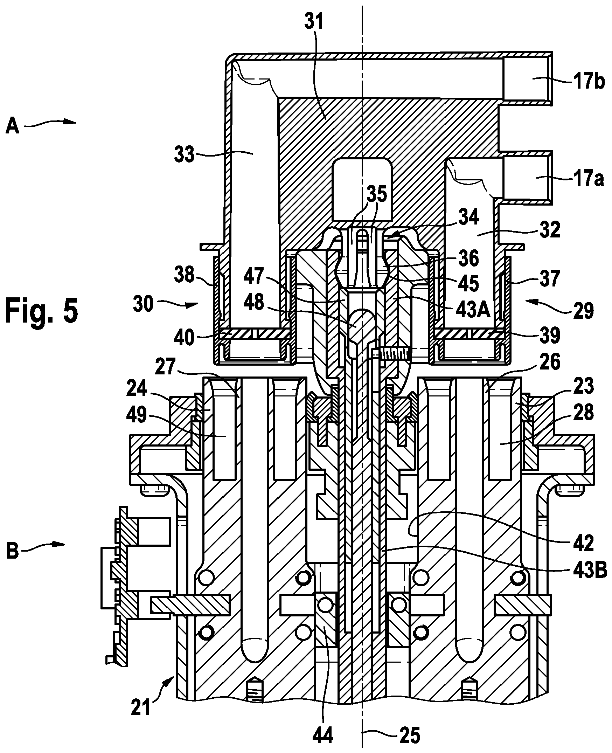

Fig. 5 zeigt die Position, in der die Stecker-Einheit A lose auf die Buchsen-Einheit B aufgesetzt ist, wobei das Ansatzstück 34 in das Aufnahmestück 43 eingerastet ist. Die Stecker-Einheit A wird dabei nur lose gehalten, ohne dass die Strömungsverbindungen hergestellt sind.

Die Stellung des Tastelements 47 wird von einer nicht dargestellten Einrichtung überwacht. Da das Tastelement 47 von dem Ansatzstück 34 zurückgeschoben ist, wird erkannt, dass die Stecker-Einheit A lose aufgesetzt ist. Wenn die Stecker-Einheit lose aufgesetzt ist, wird die Antriebseinheit in Betrieb gesetzt, wodurch der stiftförmige Körper 48 in dem Aufnahmestück 43 nach vorne geschoben wird. Dadurch wird die zunächst erst lose Verbindung zwischen Ansatzstück 34 und Aufnahmestück 43 verriegelt. Gleichzeitig werden die Anschlussteile 23, 24 mit den Anschlussstücken 26, 27 aus dem Gehäusekörper 21 nach vorne geschoben.

Mit dem Verschieben der Anschlussteile 23, 24 durchstoßen die Anschlussstücke 26, 27 die Membranen 39, 40 der Stecker-Einheit A, wodurch die flüssigkeitsdichten Verbindungen zwischen den Anschlussstücken und Konnektoren hergestellt wird. Da die Stecker-Einheit A nach der Verriegelung des Ansatzstücks mit dem Aufnahmestück fest auf der Buchsen-Einheit B sitzt, können die beim Verbinden von Stecker- und Buchsen-Einheit auftretenden Kräfte aufgenommen werden.

Das Lösen der Stecker-Einheit A von der Buchsen-Einheit B erfolgt in umgekehrter Reihenfolge. Hierzu werden der stiftförmige Körper 48 in dem Aufnahmestück 43 und die Anschlussteile 23, 24 mit den Anschlussstücken 26, 27 in dem Gehäusekörper 21 zurückgezogen, wodurch die Verbindung zwischen Ansatzstück 34 und Aufnahmestück 43 entriegelt und die Anschlussstücke 26, 27 aus den Konnektoren 29, 30 gezogen werden. Die Entriegelung kann gleichzeitig mit dem Zurückziehen der Anschlussstücke oder vor dem Zurückziehen der Anschlussstücke erfolgen.

Die Buchsen-Einheit B verfügt über einen Verschlusskörper 50 zum Verschluss der beiden Anschlussteile 23, 24, um einen Spülvorgang mit einer Spüllösung durchführen zu können.

Der Verschlusskörper 50 weist zwei Verschlussstücke 51, 52 auf, die im gleichen Abstand wie die Konnektoren 29, 30 der Stecker-Einheit A zueinander angeordnet sind und die gleiche Ausbildung aufweisen wie die Konnektoren der Stecker-Einheit aufweisen. Die beiden Verschlussstücke 51, 52 sind in dem Verschlusskörper 50 an ihrem rückwärtigen Ende verschlossen. An den beiden gegenüberliegenden Seiten, an denen die Konnektoren 29, 30 nicht angeordnet sind, weist der Verschlusskörper 50 halbkreisförmige Ausschnitte 53, 54 auf. Die Ausschnitte 53, 54 schließen mit den Verschlussstücken jeweils einen rechten Winkel ein.

Der Verschlusskörper 50 mit den Verschlussstücken 51, 52 ist mit dem vorderen Abschnitt 43A des Aufnahmestücks 43 der Buchsen-Einheit B verbunden. Da das Aufnahmestück 43 um die Längsachse 25 drehbar gelagert ist, kann durch Drehen des Aufnahmestücks 43 mit der nicht dargestellten Antriebseinheit auch der Verschlusskörper 50 mit den Verschlussstücken 51, 52 um die Achse 25 gedreht werden.

Fig. 2 zeigt den Verschlusskörper 50 mit den Verschlussstücken 51, 52 in der Position, in der sich die Stecker-Einheit A auf die Buchsen-Einheit B aufsetzen lässt. In dieser Position befinden sich die halbkreisförmigen Ausschnitte 53, 54 vor den Anschlussteilen 23, 24 bzw. Anschlussstücken 26, 27 der Buchsen-Einheit B, während die Verschlussstücke 51, 52 in einer Ebene angeordnet sind, die senkrecht auf der Ebene steht, in der die Anschlussteile 23, 24 angeordnet sind. In dieser Position kann die Stecker-Einheit A in die Buchsen-Einheit B gesteckt werden.

Für die Einleitung des Spülvorgangs wird der Verschlusskörper 50 mit den Konnektoren durch Drehen des Aufnahmestücks 43 von der nicht dargestellten Antriebseinheit um 90° verschwenkt, so dass sich die Verschlussstücke 51, 52 vor den Anschlussteilen 23, 24 befinden. Damit sind die Anschlussteile aber noch nicht verschlossen. Daraufhin werden die Anschlussteile 23, 24 aus dem Gehäusekörper 21 vorgeschoben, so dass die Verschlussstücke 51, 52 in die Anschlussteile 23, 24 eingeschoben werden. Damit sind die Spülkammern 28, 49 flüssigkeitsdicht verschlossen (Fig. 3, Fig. 7). Zur Abdichtung der Verschlussstücke 51, 52 gegenüber den Anschlussteilen 23, 24 können Ringdichtungen 55 vorgesehen sein. Nach Beendigung des Spülvorgangs werden die Anschlussteile wieder zurückgezogen. Der Verschlusskörper mit den Verschlussstücken kann nunmehr wieder in die Ausgangsposition zurückgedreht werden (Fig. 2).

Von Vorteil ist, dass nach der Ausrichtung der Verschlussstücke relativ zu den Anschlussteilen durch die Relativbewegung der Verschlussstücke und Anschlussteile beide Teile ineinandergreifen, so dass ein flüssigkeitsdichter Verschluss der Spülkammern gewähreistet ist.

Die Buchsen-Einheit B erlaubt eine vollautomatische Steuerung sowohl des Anschlusses der Stecker-Einheit A an die Buchsen-Einheit B als auch der Einleitung des Spülvorgangs, so dass die Handhabung insgesamt vereinfacht wird. Diese Steuerung erfolgt mit einer zentralen Steuereinheit, die mit der erfindungsgemäßen Positionserfassungs-Einrichtung zusammenwirkt, wobei die Positionserfassungs-Einrichtung bei diesem Ausführungsbeispiel die lineare Position der Anschlussteile 23, 24 und die Drehstellung des rohrförmigen Aufnahmestücks 43 erkennt, die nachfolgend als Antriebskörper der Antriebseinheit der Buchsen-Einheit bezeichnet werden. Die Positionserfassungs-Einrichtung zur Erfassung der linearen Position bzw. Winkelstellung einer der Antriebskörper der Antriebeinheit der medizinischen Vorrichtung wird nachfolgend unter Bezugnahme auf die Figuren 8 bis 12 in allgemeiner Form beschrieben.

Fig. 8 zeigt in schematischer Darstellung eine Anordnung von zwei Magnet-Elementen 100, 110 und einem Sensor-Element 120. Die Magnet-Elemente 100, 110 sind vorzugsweise Permanentmagnete, die beispielsweise an einem nicht dargestellten längsverschiebbaren Antriebskörper angeordnet sind, dessen lineare Position erfasst werden soll. Das Sensor-Element 120 ist vorzugsweise ein Hall-Sensor, der ortsfest an einem nicht dargestellten Gehäuseteil angeordnet ist.Die Magnet-Elemente 100, 110 sind derart im Abstand zueinander angeordnet, dass deren Pole N und S in entgegengesetzte Richtungen weisen. Es sei angenommen, dass die Magnet-Elemente 100, 110, die im Abstand zu dem Sensor-Element 120 angeordnet sind, entlang einer parallel zu den Magneten verlaufenden Achse um die Wegstrecke Δx verschoben werden können. Die Magnet-Elemente 100, 110 können aber auch ortsfest an einem nicht dargestellten Gehäuseteil angeordnet sein, während das Sensor-Element 120 an einem nicht dargestellten längsverschiebbaren Antriebskörper angeordnet ist, dessen lineare Position erfasst werden soll.

Der Sensor 120 erzeugt ein elektrisches Signal, das mit der magnetischen Flussdichte B korreliert. Es zeigt sich, dass der Betrag der magnetischen Flussdichte B auf der Höhe der durch Nord- und Südpol N, S der Magnete 100, 110 verlaufenden Achse 105, 115 maximal bzw. minimal ist und zu beiden Seiten abnimmt (Positionen A und B). Dabei ergibt sich ein sinusförmiger Verlauf. Weiterhin zeigt sich, dass beispielsweise in den Positionen C und D oder allen anderen Positionen zu beiden Seiten der Symmetrieachse die magnetische Flussdichte B den gleichen Betrag hat, obwohl sich der Sensor an unterschiedlichen Positionen befindet, d. h. in Richtung der x-Achse betrachtet vor oder hinter dem ersten Magneten 100 liegt. Eine eindeutige Bestimmung der linearen Position des Sensors bzw. des Antriebskörpers ist daher mit der vorliegenden Messanordnung nicht möglich. Zu dem gleichen Ergebnis kommt man, wenn man mit einer vergleichbaren Messanordnung eine rotatorische Bewegung des Antriebskörpers erfasst.

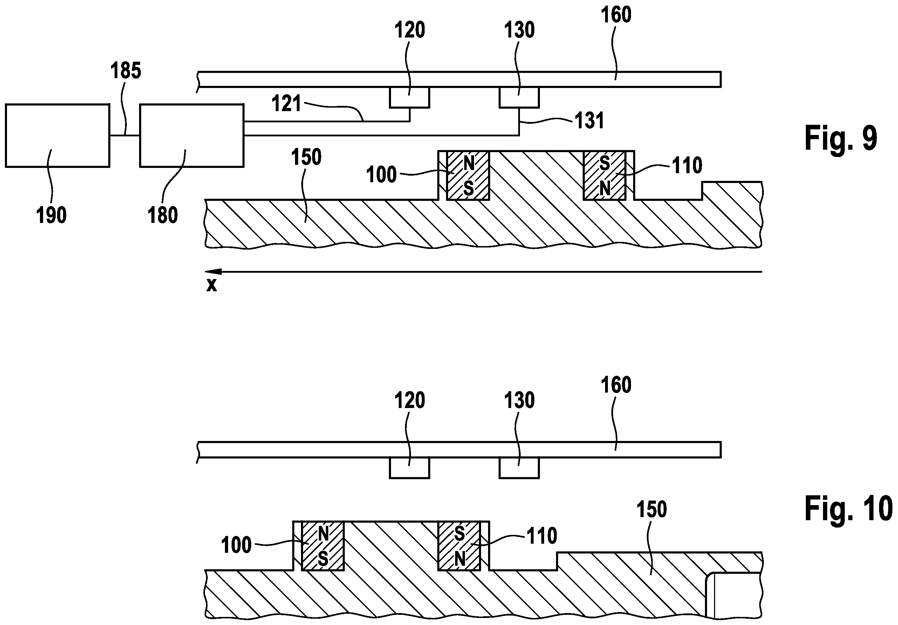

Fig. 9 und Fig. 10 zeigt eine erste Ausführungsform der erfindungsgemäßen Positionserfassungs-Einrichtung, mit der die lineare Position eines Antriebskörpers der Antriebseinheit der Buchsen-Einheit erkannt wird.

Die Positionserfassungs-Einrichtung verfügt über zwei Magnet-Elemente 100, 110, insbesondere Permanentmagnete, die an dem längsverschiebbaren Antriebskörper 150, insbesondere den längsverschiebbaren Anschlussteilen 23, 24 (Fig. 4) im Abstand zueinander angeordnet sind, wobei die Pole N, S der Magnete 100, 110 in entgegengesetzte Richtungen zeigen. An einem Gehäuseteil 160 der Buchsen-Einheit sind den Magneten gegenüberliegend zwei Sensor-Elemente 120, 130, insbesondere Hall-Sensoren, im Abstand zueinander angeordnet. Die Hall-Sensoren 120, 130 erzeugen ein elektrisches Signal, das mit der magnetischen Flussdichte korreliert. Darüber hinaus kann die Positionserfassungs-Einrichtung eine Auswerteinheit 180 aufweisen, die über eine Datenleitung 185 mit einer Steuereinheit 190 verbunden ist, mit der die nicht dargestellten Stellantriebe der Antriebseinheit der Buchsen-Einheit gesteuert werden. Die Auswerteinheit 180 empfängt über Signalleitungen 121, 131 die elektrischen Signale der Hall-Sensoren 120, 130. Die Auswerteinheit 180 kann für die Steuereinheit 190 ein erstes Steuersignal erzeugen, wenn sich der Antriebskörper 150 in der vorgeschobenen Position (Fig. 10) befindet, und ein zweites Steuersignal, wenn sich der Antriebskörper in der zurückgezogenen Position (Fig. 9) befindet.
Die Auswerteinheit 180, die ein Mikroprozessor sein kann, auf dem ein Datenverarbeitungsprogramm (Software) läuft, ist derart konfiguriert, dass auf der Grundlage der Amplitude des Signals des ersten Hall-Sensors 120 die beiden möglichen linearen Positionen des Antriebskörpers 150 in Bezug auf die in Fig. 8 gezeigte Symmetrieachse 105 bzw. 115 des Magneten ermittelt wird. Auf der Grundlage des Vorzeichens des Signals des zweiten Hall-Sensors 130 bestimmt die Auswerteinheit 180, ob die mit dem ersten Sensor 120 ermittelte lineare Position in Richtung der X-Achse betrachtet die Position vor oder hinter der Symmetrieachse 105 bzw. 115 ist. Wenn beispielsweise das Vorzeichen des Signals des zweiten Sensors 130 positiv ist, wird darauf geschlossen, dass sich erste Sensor 120 vor der Symmetrieachse und der Antriebskörper 150 in der vorgeschobenen Position befindet (Fig. 10), und bei einem negativen Vorzeichen wird darauf geschlossen, dass sich der erste Sensor 120 hinter der Symmetrieachse 105 und der Antriebskörper 150 in der zurückgezogenen Position befindet (Fig. 9). Die beiden Magnete 120, 130 können auch an dem beweglichen Antriebskörper 150 und die beiden Sensoren an dem ortfesten Gehäuseteil 160 angeordnet sein.

Die Steuereinheit 190 kann eine kontinuierliche Positionserfassung für die elektrische Regelung der Stellantriebe vorsehen, wobei mit der Verarbeitung der Steuersignale der Auswerteinheit 180 eine eindeutige Positionsbestimmung möglich ist. Die Aufteilung in eine Auswerteinheit und eine Steuereinheit soll nur der Veranschaulichung der Erfindung dienen. In der Praxis werden aber Steuer- und Auswerteinheit eine gemeinsame Einheit sein, die von einem Mikrokontroller gebildet wird.

Zur Erfassung der Winkelstellung des Antriebskörpers der Antriebseinheit kann die Positionserfassungs-Einrichtung zwei Magnet-Elemente und zwei Sensor-Elemente aufweisen, die in einer senkrecht auf der Achse des Antriebskörpers stehenden Ebene an dem Antriebskörper bzw. dem Gehäuseteil angeordnet sind. Die Auswertung der Signale für die Bestimmung der Winkelposition des Antriebskörpers erfolgt analog zu der Signalauswertung für die Bestimmung der linearen Position.

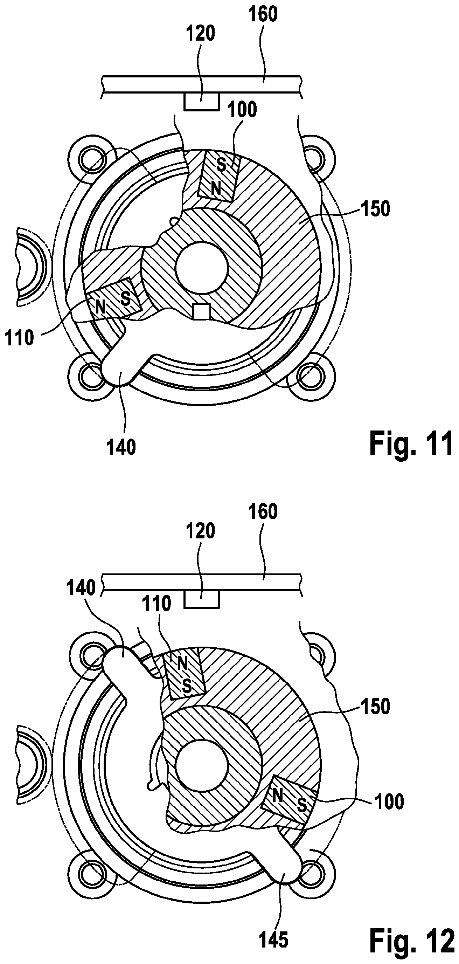

Die Figuren 11 und 12 zeigen in teilweiser geschnittene Darstellung eine alternative Ausführungsform der Positionserfassungs-Einrichtung, die zur Bestimmung der Winkelstellung des Antriebskörpers dient. Diese Einrichtung unterscheidet sich von der Positionserfassungs-Einrichtung von den Figuren 9 und 10 dadurch, dass sie über nur ein Sensor-Element 120 verfügt, das an dem Gehäuseteil 160 befestigt ist. Die einander entsprechenden Teile sind wieder mit den gleichen Bezugszeichen versehen.

Die in den Figuren 11 und 12 nicht dargestellte Auswerteinheit ist derart konfiguriert, dass auf der Grundlage der Amplitude des Signals des einzigen Hall-Sensors 120 die beiden möglichen Winkelstellung des Antriebskörpers 150 in Bezug auf die in Fig. 8 gezeigte Symmetrieachse des Magneten ermittelt wird, d. h. eine Winkelstellung vor bzw. hinter der Symmetrieachse. Weiterhin bestimmt die Auswerteinheit das Vorzeichen des Gradienten des von dem einzigen Hall-Sensors 120 erfassten Magnetfeldes bei einer Änderung der Winkelstellung und ermittelt auf der Grundlage des Vorzeichens des Gradienten, ob die mit dem ersten Hall-Sensor 120 bestimmte Winkelstellung vor oder hinter der Symmetrieachse liegt.

Fig. 8 zeigt für den analogen Fall einer translatorischen Bewegung des Sensors, dass mit dem Vorzeichen des Gradienten die Winkelstellung eindeutig festgelegt ist. Es sei angenommen, dass für die Position C und D die Amplitude des Messsignals B₁ ist. Damit ist die Position aber nicht eindeutig festgelegt. Wenn der Gradient der magnetischen Flussdichte beispielsweise negativ ist, d. h. die Steigung der sinusförmigen Kurve negativ ist, wird darauf geschlossen, dass sich der Sensor in der Position D befindet, so dass die Auswerteinheit das erste Steuersignal erzeugt, während bei einem positiven Gradienten, d. h. die Steigung der Kurve positiv ist, darauf geschlossen wird, dass sich der Sensor in der Position C befindet, so dass die Auswerteinheit das zweite Steuersignal erzeugt.

Für die Bestimmung des Vorzeichens des Gradienten ist eine Änderung der Winkelstellung bzw. der linearen Position um nur einen kleinen Betrag ausreichend. Das Vorzeichen des Gradienten, d. h. ein Zunahme bzw. Abnahme der magnetischen Flussdichte, kann daher nur für die eigentliche Messung erfolgen. Es ist aber auch möglich, das Vorzeichen des Gradienten dann zu bestimmen, wenn der Antriebskörper ohnehin verschoben oder gedreht werden muss.

Eine weitere alternative Ausführungsform mit nur einem Sensor-Element 120 sieht vor, einen Messbereich vorzugeben, in dem eine eindeutige Bestimmung der Position möglich ist. Dieser Messbereich ist der Bereich, der in Fig. 8 zwischen den beiden Symmetrieachsen 105, 115 liegt. Bei der alternativen Ausführungsform sind daher zwei Anschläge vorgesehen, die den Bewegungsbereich des Antriebskörpers 150 in beiden Richtungen begrenzen. Diese Anschläge 140, 145 sind in den Figuren 11 und 12 nur andeutungsweise dargestellt. In Fig. 11 ist die Winkelstellung gezeigt, in der der Antriebskörper 150 an dem einen Anschlag anschlägt, und in Fig. 12 die Winkelstellung, in der der der Antriebskörper an dem anderen Anschlag anschlägt. Der Drehwinkel ist hierbei derart bemessen, dass sich die Magnete 100, 110 nicht über die Symmetrieachsen hinaus in Positionen bewegen können, an denen sich mit einem einzigen Sensor die Winkelstellung des Antriebskörpers nicht eindeutig bestimmen ließe.

Die erfindungsgemäße Positionserfassung-Einrichtung stellt ein einfaches, aber zuverlässiges Messsystem dar, mit dem die Steuerung der medizinischen Vorrichtung verbessert werden kann. Die Steuerung der Vorrichtung kann mit der Steuereinheit 190 auf der Grundlage der Steuersignale der Auswerteinheit 180 erfolgen.

Die eindeutige Bestimmung der Position mit zwei oder mehr Sensor-Elementen außerhalb eines Messbereichs, der eine eindeutige Bestimmung mit nur einem Sensor-Element nicht zulässt, erlaubt eine Anordnung der einzelnen Elemente, in der sich eine hohe Auflösung und eine hohe Messgenauigkeit erzielen lässt.

## Patentansprüche

1. Medizinische Vorrichtung mit einer Buchsen-Einheit (B) zum Anschluss einer Stecker-Einheit einer Vorrichtung zur Bereitstellung von medizinischen Flüssigkeiten,
wobei die Buchsen-Einheit (B) mindestens ein Anschlussstück (27, 28) zum Anschluss mindestens eines Konnektors der Stecker-Einheit aufweist, so dass durch den Anschluss des Konnektors an das Anschlussstück eine Strömungsverbindung zwischen der medizinischen Vorrichtung und der Vorrichtung zur Bereitstellung von medizinischen Flüssigkeiten zum Zuführen frischer oder Abführen gebrauchter Flüssigkeit herstellbar ist,
**dadurch gekennzeichnet, dass**
die Buchsen-Einheit (B) eine Anschluss-Einrichtung zur Herstellung einer Verbindung zwischen dem mindestens einen Anschlussstück (27, 28) und dem mindestens einen Konnektor aufweist, wobei die Anschluss-Einrichtung eine Antriebs-Einheit aufweist, die mindestens einen beweglichen Antriebskörper (150) aufweist, der eine translatorische oder rotatorische Bewegung ausführt,
mindestens eine Positionserfassungs-Einrichtung zur Erfassung der linearen Position oder der Winkelstellung des Antriebskörpers (150) vorgesehen ist, und die Positionserfassungs-Einrichtung aufweist:
mindestens zwei im Abstand zueinander angeordnete ein Magnetfeld erzeugende Magnet-Elemente (100, 110) und mindestens zwei das Magnetfeld der Magnet-Elemente erfassende Sensor-Elemente (120, 130), wobei die mindestens zwei Magnet-Elemente an dem beweglichen Antriebskörper und die mindestens zwei Sensor-Elemente ortsfest gegenüber dem beweglichen Antriebskörper oder die mindestens zwei Magnet-Elemente gegenüber dem beweglichen Antriebskörper ortsfest und die mindestens zwei Sensor-Elemente an dem beweglichen Antriebskörper angeordnet sind, und
eine Auswerteinheit (180), die derart konfiguriert ist, dass die lineare Position oder Winkelstellung des Antriebskörpers (150) auf der Grundlage des von den mindestens zwei Magnet-Elementen (100, 110) erzeugten und von den mindestens zwei Sensor-Elementen (120, 130) erfassten Magnetfeldes bestimmt wird, wobei
die mindestens zwei Magnet-Elemente (100, 110) der Positionserfassungs-Einrichtung mindestens
ein erstes Magnet-Element (100) und ein zweites Magnet-Element (110) sind, die im Abstand zueinander mit einander entgegengesetzter Polarität angeordnet sind, und die mindestens zwei Sensor-Elemente (120, 130) der Positionserfassungs-Einrichtung mindestens ein erstes Sensor-Element (120) und ein zweites Sensor-Element (130) sind, die im Abstand zueinander angeordnet sind, wobei die Auswerteinheit (180) derart konfiguriert ist, dass auf der Grundlage des von dem ersten Sensor-Element (120) erfassten Magnetfeldes zwei mögliche lineare Positionen oder Winkelstellungen des Antriebskörpers (150) bestimmt werden und auf der Grundlage des von dem zweiten Sensor-Element (130) erfassten Magnetfeldes bestimmt wird, ob die mit dem ersten Sensor-Element bestimmte lineare Position oder Winkelstellung die erste oder zweite Position bzw. Winkelstellung der zwei möglichen Positionen bzw. Winkelstellungen ist.

2. Medizinische Vorrichtung nach Anspruch 1, **dadurch gekennzeichnet, dass** die Auswerteinheit (180) derart konfiguriert ist, dass auf der Grundlage des Vorzeichens des von dem zweiten Sensor-Element (130) erfassten Magnetfeldes bestimmt wird, ob die mit dem ersten Sensor-Element (120) bestimmte lineare Position bzw. Winkelstellung die erste oder zweite Position bzw. Winkelstellung der zwei möglichen Positionen bzw. Winkelstellungen ist.

3. Medizinische Vorrichtung mit einer Buchsen-Einheit (B) zum Anschluss einer Stecker-Einheit einer Vorrichtung zur Bereitstellung von medizinischen Flüssigkeiten,
wobei die Buchsen-Einheit (B) mindestens ein Anschlussstück (27, 28) zum Anschluss mindestens eines Konnektors der Stecker-Einheit aufweist, so dass durch den Anschluss des Konnektors an das Anschlussstück eine Strömungsverbindung zwischen der medizinischen Vorrichtung und der Vorrichtung zur Bereitstellung von medizinischen Flüssigkeiten zum Zuführen frischer oder Abführen gebrauchter Flüssigkeit herstellbar ist,
**dadurch gekennzeichnet, dass**
die Buchsen-Einheit (B) eine Anschluss-Einrichtung zur Herstellung einer Verbindung zwischen dem mindestens einen Anschlussstück (27, 28) und dem mindestens einen Konnektor aufweist, wobei die Anschluss-Einrichtung eine Antriebs-Einheit aufweist, die mindestens einen beweglichen Antriebskörper (150) aufweist, der eine translatorische Bewegung ausführt,
mindestens eine Positionserfassungs-Einrichtung zur Erfassung der linearen Position des Antriebskörpers (150) vorgesehen ist, wobei
die Positionserfassungs-Einrichtung aufweist:
mindestens zwei im Abstand zueinander angeordnete ein Magnetfeld erzeugende Magnet-Elemente (100, 110) und mindestens ein das Magnetfeld der Magnet-Elemente erfassendes Sensor-Element (120), wobei die mindestens zwei Magnet-Elemente an dem beweglichen Antriebskörper und das mindestens eine Sensor-Element ortsfest gegenüber dem beweglichen Antriebskörper oder die mindestens zwei Magnet-Elemente gegenüber dem beweglichen Antriebskörper ortsfest und das mindestens eine Sensor-Element an dem beweglichen Antriebskörper angeordnet sind, und
eine Auswerteinheit (180), die derart konfiguriert ist, dass die lineare Position des Antriebskörpers (150) auf der Grundlage des von den mindestens zwei Magnet-Elementen (100, 110) erzeugten und von dem mindestens einen Sensor-Element (120) erfassten Magnetfeldes bestimmt wird, wobei
die translatorische Bewegung des Antriebskörpers (150) durch ein oder mehrere Anschlagelemente (140, 145) begrenzt ist.

4. Medizinische Vorrichtung mit einer Buchsen-Einheit (B) zum Anschluss einer Stecker-Einheit einer Vorrichtung zur Bereitstellung von medizinischen Flüssigkeiten,
wobei die Buchsen-Einheit (B) mindestens ein Anschlussstück (27, 28) zum Anschluss mindestens eines Konnektors der Stecker-Einheit aufweist, so dass durch den Anschluss des Konnektors an das Anschlussstück eine Strömungsverbindung zwischen der medizinischen Vorrichtung und der Vorrichtung zur Bereitstellung von medizinischen Flüssigkeiten zum Zuführen frischer oder Abführen gebrauchter Flüssigkeit herstellbar ist,
**dadurch gekennzeichnet, dass**
die Buchsen-Einheit (B) eine Anschluss-Einrichtung zur Herstellung einer Verbindung zwischen dem mindestens einen Anschlussstück (27, 28) und dem mindestens einen Konnektor aufweist, wobei die Anschluss-Einrichtung eine Antriebs-Einheit aufweist, die mindestens einen beweglichen Antriebskörper (150) aufweist, der eine rotatorische Bewegung ausführt,
mindestens eine Positionserfassungs-Einrichtung zur Erfassung der Winkelstellung des Antriebskörpers (150) vorgesehen ist, wobei
die Positionserfassungs-Einrichtung aufweist:
mindestens zwei im Abstand zueinander angeordnete ein Magnetfeld erzeugende Magnet-Elemente (100, 110) und mindestens ein das Magnetfeld der Magnet-Elemente erfassendes Sensor-Element (120), wobei die mindestens zwei Magnet-Elemente an dem beweglichen Antriebskörper und das mindestens eine Sensor-Element ortsfest gegenüber dem beweglichen Antriebskörper oder die mindestens zwei Magnet-Elemente gegenüber dem beweglichen Antriebskörper ortsfest und das mindestens eine Sensor-Element an dem beweglichen Antriebskörper angeordnet sind, und
eine Auswerteinheit (180), die derart konfiguriert ist, dass die Winkelstellung des Antriebskörpers (150) auf der Grundlage des von den mindestens zwei Magnet-Elementen (100, 110) erzeugten und von dem mindestens einen Sensor-Element (120) erfassten Magnetfeldes bestimmt wird, wobei
die rotatorische Bewegung des Antriebskörpers (150) durch ein oder mehrere Anschlagelemente (140, 145) begrenzt ist.

5. Medizinische Vorrichtung mit einer Buchsen-Einheit (B) zum Anschluss einer Stecker-Einheit einer Vorrichtung zur Bereitstellung von medizinischen Flüssigkeiten,
wobei die Buchsen-Einheit (B) mindestens ein Anschlussstück (27, 28) zum Anschluss mindestens eines Konnektors der Stecker-Einheit aufweist, so dass durch den Anschluss des Konnektors an das Anschlussstück eine Strömungsverbindung zwischen der medizinischen Vorrichtung und der Vorrichtung zur Bereitstellung von medizinischen Flüssigkeiten zum Zuführen frischer oder Abführen gebrauchter Flüssigkeit herstellbar ist,
**dadurch gekennzeichnet, dass**
die Buchsen-Einheit (B) eine Anschluss-Einrichtung zur Herstellung einer Verbindung zwischen dem mindestens einen Anschlussstück (27, 28) und dem mindestens einen Konnektor aufweist, wobei die Anschluss-Einrichtung eine Antriebs-Einheit aufweist, die mindestens einen beweglichen Antriebskörper (150) aufweist, der eine translatorische oder rotatorische Bewegung ausführt,
mindestens eine Positionserfassungs-Einrichtung zur Erfassung der linearen Position oder der Winkelstellung des Antriebskörpers (150) vorgesehen ist, wobei die Positionserfassungs-Einrichtung aufweist:
mindestens zwei im Abstand zueinander angeordnete ein Magnetfeld erzeugende Magnet-Elemente (100, 110) und mindestens ein das Magnetfeld der Magnet-Elemente erfassendes Sensor-Element (120), wobei die mindestens zwei Magnet-Elemente an dem beweglichen Antriebskörper und das mindestens eine Sensor-Element ortsfest gegenüber dem beweglichen Antriebskörper oder die mindestens zwei Magnet-Elemente gegenüber dem beweglichen Antriebskörper ortsfest und das mindestens eine Sensor-Element an dem beweglichen Antriebskörper angeordnet sind, und
eine Auswerteinheit (180), die derart konfiguriert ist, dass die lineare Position des Antriebskörpers (150) auf der Grundlage des von den mindestens zwei Magnet-Elementen (100, 110) erzeugten und von dem mindestens einen Sensor-Element (120) erfassten Magnetfeldes bestimmt wird, wobei
die mindestens zwei Magnet-Elemente (100, 110) der Positionserfassungs-Einrichtung mindestens
ein erstes Magnet-Element (100) und ein zweites Magnet-Element (110) sind, die im Abstand zueinander mit einander entgegengesetzter Polarität angeordnet sind, wobei die Auswerteinheit (180) derart konfiguriert ist, dass auf der Grundlage des von dem mindestens einen Sensor-Element (120) erfassten Magnetfeldes zwei mögliche lineare Positionen und/oder Winkelstellungen des Antriebskörpers bestimmt werden, und dass die Auswerteinheit (180) derart konfiguriert ist, dass der Gradient der Änderung des von dem Sensor-Element (120) erfassten Magnetfeldes bei einer Änderung der linearen Position bzw. Winkelstellung des Antriebskörpers bestimmt wird, und auf der Grundlage des Vorzeichens des Gradienten der Änderung bestimmt wird, ob die mit dem ersten Sensor-Element bestimmte lineare Position bzw. Winkelstellung die erste oder zweite Position bzw. Winkelstellung der zwei möglichen Positionen bzw. Winkelstellungen ist.

6. Medizinische Vorrichtung nach einem der Ansprüche 1 bis 5, **dadurch gekennzeichnet, dass** die Antriebs-Einheit der Anschluss-Einrichtung einen Antriebskörper (150) aufweist, der Mittel zum lösbaren Verbinden der Stecker-Einheit mit der Buchsen-Einheit aufweist.

7. Medizinische Vorrichtung nach Anspruch 6, **dadurch gekennzeichnet, dass** die Mittel zum lösbaren Verbinden der Stecker-Einheit mit der Buchsen-Einheit ein Aufnahmestück (43) zum Einstecken eines Ansatzstückes der Stecker-Einheit aufweisen.

8. Medizinische Vorrichtung nach einem der Ansprüche 1 bis 7, **dadurch gekennzeichnet, dass** die Auswerteinheit (180) ein erstes Steuersignal erzeugt, wenn die Positionserfassungs-Einrichtung eine erste vorgeschobene Position des Antriebskörpers (150) detektiert, in der der mindestens eine Konnektor und das mindestens eine Anschlussstück getrennt sind, und ein zweites Steuersignal erzeugt, wenn die Positionserfassungs-Einrichtung eine zweite zurückgezogene Stellung des Antriebskörpers (150) detektiert, in der der mindestens eine Konnektor an das mindestens eine Anschlussstück angeschlossen ist.

9. Medizinische Vorrichtung nach einem der Ansprüche 1 bis 5, **dadurch gekennzeichnet, dass** der Antriebskörper (150) der Antriebseinheit der Anschluss-Einrichtung um eine Drehachse (25) drehbar ist.

10. Medizinische Vorrichtung nach Anspruch 9, **dadurch gekennzeichnet, dass** das mindestens eine Anschlussstück (26, 27) der Buchsen-Einheit (B) unter Bildung einer Spülkammer (28, 49) von einem Anschlussteil (23, 24) konzentrisch umschlossen ist und die Buchsen-Einheit (B) einen Verschlusskörper (50) mit mindestens einem Verschlussstück (51, 52) zum Verschluss der mindestens einen Spülkammer aufweist, wobei
der Verschlusskörper (50) an dem drehbaren Antriebskörper vorgesehen ist, und das mindestens eine Verschlussstück (51, 52) an dem Verschlusskörper (50) im Abstand zu der Drehachse angeordnet ist, und
in einer ersten Drehstellung das mindestens eine Verschlussstück (51, 52) und der mindestens eine Anschlussteil (23, 24) auf einer gemeinsamen Achse liegen, so dass durch eine Relativbewegung von Stecker-Einheit und Buchsen-Einheit (B) eine Verbindung zwischen Verschlussstück und Anschlussteil zum Verschluss der Spülkammer herstellbar ist, und
in einer zweiten Drehstellung das mindestens eine Verschlussstück (51, 52) und das mindestens eine Anschlussstück (23, 24) zueinander versetzt angeordnet sind, so dass beim Einstecken der Stecker-Einheit in die Buchsen-Einheit (B) eine Verbindung zwischen dem mindestens einen Konnektor (29, 30) der Stecker-Einheit (A) und dem mindestens einen Anschlussstück (26, 27) der Buchsen-Einheit (B) herstellbar ist.

11. Medizinische Vorrichtung nach Anspruch 10, **dadurch gekennzeichnet, dass** die Auswerteinheit (180) ein erstes Steuersignal erzeugt, wenn die Positionserfassungs-Einrichtung die erste Drehstellung detektiert und ein zweites Steuersignal erzeugt, wenn die Positionserfassungs-Einrichtung die zweite Drehstellung detektiert.

12. Medizinische Vorrichtung nach einem der Ansprüche 1 bis 11, **dadurch gekennzeichnet, dass** das Magnet-Element (100, 110) ein Permanentmagnet ist.

13. Medizinische Vorrichtung nach einem der Ansprüche 1 bis 12, **dadurch gekennzeichnet, dass** das Sensor-Element (120) ein Hall-Sensor ist.

14. Medizinische Vorrichtung nach einem der Ansprüche 1 bis 13, **dadurch gekennzeichnet, dass** die medizinische Vorrichtung eine Blutbehandlungsvorrichtung (2), insbesondere eine extrakorporale Dialysevorrichtung oder eine Vorrichtung zur Peritionealdialyse ist.

## Claims

1. A medical device comprising a socket unit (B) for connecting a plug unit of a device for providing medical fluids,
wherein the socket unit (B) has at least one connecting piece for connecting at least one connector of the plug unit so that when connecting the connector to the connecting piece, a flow connection for feeding fresh fluids or discharging used fluids between the medical device and the device for providing medical fluids can be established,
**characterized in that**
the socket unit (B) has a connecting mechanism for establishing a connection between the at least one connecting piece (27, 28) and the at least one connector, wherein the connecting mechanism has a drive unit that has at least one movable drive body (150) that carries out a translational or rotational movement,
at least one position detection mechanism for detecting the linear position or the angular position of the drive body (150) is provided, wherein
the position detection mechanism comprises:
at least two magnet elements (100, 110) that are arranged spaced apart from each other and generate a magnetic field, and at least two sensor elements (120, 130) that detect the magnetic field of the magnet elements, wherein the at least two magnet elements are arranged on the movable drive body and the at least two sensor elements are arranged stationarily in relation to the movable drive body, or the at least two magnet elements are arranged stationarily in relation to the movable drive body and the at least two sensor elements are arranged on the movable drive body, and
an evaluation unit that is configured such that the linear position or the angular position of the drive body (150) is determined based on the magnetic field generated by the at least two magnet elements (100, 110) and detected by the at least two sensor elements (120, 130), wherein the at least two magnetic elements (100, 110) of the position detection mechanism are at least a first magnet element (100) and a second magnet element (110) that are arranged spaced apart from each other with opposite polarity, and the at least two sensor elements (120, 130) of the position detection mechanism are at least a first sensor element (120) and a second sensor element (130) that are spaced apart from each other, wherein the evaluation unit (180) is configured such that based on the magnetic field detected by the first sensor element (120), two possible linear positions or angular positions of the drive body (150) are determined, and based of the magnetic field detected by the second sensor element (130), it is determined whether the linear position or angular position determined with the first sensor element is the first or the second linear position and/or angular position of the two possible positions and/or angular positions.

2. The medical device according to claim 1, **characterized in that** the evaluation unit (180) is configured such that based on the sign of the magnetic field detected by the second sensor element (130) it is determined whether the linear position and/or angular position detected by the first sensor element (120) is the first or second position and/or angular position of the two possible positions and/or angular positions.

3. A medical device comprising a socket unit (B) for connecting a plug unit of a device for providing medical fluids,
wherein the socket unit (B) has at least one connecting piece (27, 28) for connecting at least one connector of the plug unit so that when connecting the connector to the connecting piece, a flow connection for feeding fresh fluids or discharging used fluids between the medical device and the device for providing medical fluids can be established,
**characterized in that**
the socket unit (B) has a connecting mechanism for establishing a connection between the at least one connecting piece (27, 28) and the at least one connector, wherein the connecting mechanism has a drive unit that has at least one movable drive body (150) that carries out a translational movement,
at least one position detection mechanism for detecting the linear position of the drive body (150) is provided, wherein
the position detection mechanism comprises:
at least two magnet elements (100, 110) that are arranged spaced apart from each other and generate a magnetic field, and at least one sensor element (120) that detects the magnetic field of the magnet elements, wherein the at least two magnet elements are arranged on the movable drive body and the at least one sensor element is arranged stationarily in relation to the movable drive body, or the at least two magnet elements are arranged stationarily in relation to the movable drive body and the at least one sensor element is arranged on the movable drive body, and
an evaluation unit (180) that is configured such that the linear position of the drive body (150) is determined based on the magnetic field generated by the at least two magnet elements (100, 110) and detected by the at least one sensor element (120), wherein the translational movement of the drive body (150) is limited by one or a plurality of stop elements (140, 145).

4. A medical device comprising a socket unit (B) for connecting a plug unit of a device for providing medical fluids,
wherein the socket unit (B) has at least one connecting piece (27, 28) for connecting at least one connector of the plug unit so that when connecting the connector to the connecting piece, a flow connection for feeding fresh fluids or discharging used fluids between the medical device and the device for providing medical fluids can be established,
**characterized in that**
the socket unit (B) has a connecting mechanism for establishing a connection between the at least one connecting piece (27, 28) and the at least one connector, wherein the connecting mechanism has a drive unit that has at least one movable drive body (150) that carries out a rotational movement,
at least one position detection mechanism for detecting the angular position of the drive body (150) is provided, wherein
the position detection mechanism comprises:
at least two magnet elements (100, 110) that are arranged spaced apart from each other and generate a magnetic field, and at least one sensor element (120) that detects the magnetic field of the magnet elements, wherein the at least two magnet elements are arranged on the movable drive body and the at least one sensor element is arranged stationarily in relation to the movable drive body, or the at least two magnet elements are arranged stationarily in relation to the movable drive body and the at least one sensor element is arranged on the movable drive body, and
an evaluation unit (180) that is configured such that the angular position of the drive body (150) is determined based on the magnetic field generated by the at least two magnet elements (100, 110) and detected by the at least one sensor element (120), wherein the rotational movement of the drive body (150) is limited by one or a plurality of stop elements (140, 145).

5. A medical device comprising a socket unit (B) for connecting a plug unit of a device for providing medical fluids,
wherein the socket unit (B) has at least one connecting piece (27, 28) for connecting at least one connector of the plug unit so that when connecting the connector to the connecting piece, a flow connection for feeding fresh fluids or discharging used fluids between the medical device and the device for providing medical fluids can be established,
**characterized in that**
the socket unit (B) has a connecting mechanism for establishing a connection between the at least one connecting piece (27, 28) and the at least one connector, wherein the connecting mechanism has a drive unit that has at least one movable drive body (150) that carries out a translational or rotational movement,
at least one position detection mechanism for detecting the linear position or the angular position of the drive body (150) is provided, wherein
the position detection mechanism comprises:
at least two magnet elements (100, 110) that are arranged spaced apart from each other and generate a magnetic field, and at least one sensor element (120) that detects the magnetic field of the magnet elements, wherein the at least two magnet elements are arranged on the movable drive body and the at least one sensor element is arranged stationarily in relation to the movable drive body, or the at least two magnet elements are arranged stationarily in relation to the movable drive body and the at least one sensor element is arranged on the movable drive body, and
an evaluation unit (180) that is configured such that the linear position of the drive body (150) is determined based on the magnetic field generated by the at least two magnet elements (100, 110) and detected by the at least one sensor element (120), wherein the at least-two magnetic elements (100, 110) of the position detection mechanism are at least a first magnet element (100) and a second magnet element (110) that are arranged spaced apart from each with opposite polarity, wherein the evaluation unit (180) is configured such that based on the magnetic field detected by the at least one sensor element (120), two possible linear positions and/or angular positions of the drive body are determined, and that the evaluation unit (180) is configured such that the gradient of the change of the magnetic field detected by the sensor element (120) is determined in the case of a change of the linear position and/or the angular position of the drive body, and based on the sign of the gradient of the change it is determined whether the linear position and/or angular position determined with the first sensor element is the first or second position and/or angular position of the two possible positions and/or angular positions.

6. The medical device according to any one of the claims 1 to 5, **characterized in that** the drive unit of the connecting mechanism has a drive body (150) that has means for detachably connecting the plug unit to the socket unit.

7. The medical device according to claim 6, **characterized in that** the means for detachably connecting the plug unit to the socket unit have a receptacle (43) for plugging in an attachment piece of the plug unit.

8. The medical device according to any one of the claims 1 to 7, **characterized in that** the evaluation unit (180) generates a first control signal when the position detection mechanism detects a first advanced position of the drive body (150) in which the at least one connector and the at least one connecting piece are disconnected, and generates a second control signal when the position detection mechanism detects a second retracted position of the drive body (150) in which the at least one connector is connected to the at least one connecting piece.

9. The medical device according to any one of the claims 1 to 5, **characterized in that** the drive body (150) of the drive unit of the connecting device is pivotable about a pivot axis (25).

10. The medical device according to claim 9, **characterized in that** the at least one connecting piece (26, 27) of the socket unit (B) is concentrically enclosed by a connecting part (23, 24) thereby forming a rinsing chamber (28, 49), and the socket unit (B) has a closure body (50) with at least one closure piece (51, 52) for closing the at least one rinsing chamber, wherein
the closure body (50) is provided on the pivotable drive body, and the at least one closure piece (51, 52) is arranged on the closure body (50) and is spaced apart from the pivot axis, and
in a first pivot position, the at least one closure piece (51, 52) and the at least one connecting part (23, 24) lie on a common axis so that through a relative movement of plug unit and socket unit (B), a connection between closure piece and connecting piece can be established for closing the rinsing chamber, and
in a second pivot position, the at least one closure piece (51, 52) and the at least one connecting piece (23, 24) are arranged offset to each other so that when plugging the plug unit into the socket (B), a connection between the at least one connector (29, 30) of the plug unit (A) and the at least one connecting piece (26, 27) of the socket unit (B) can be established.

11. The medical device according to claim 10, **characterized in that** the evaluation unit (180) generates a first control signal when the position detection mechanism detects the first pivot position, and generates a second control signal when the position detection mechanism detects the second pivot position.

12. The medical device according to any one of the claims 1 to 11, **characterized in that** the magnet element (100, 110) is a permanent magnet.

13. The medical device according to any one of the claims 1 to 12, **characterized in that** the sensor element (120) is a Hall sensor.

14. The medical device according to any one of the claims 1 to 13, **characterized in that** the medical device is a blood treatment device (2), in particular an extra-corporal dialysis device, or a device for peritoneal dialysis.

## Revendications

1. Dispositif médical avec une unité douille (B) pour le raccordement d'une unité fiche d'un dispositif pour la mise à disposition de liquides médicaux,
où l'unité douille (B) présente au moins une pièce de raccordement (27, 28) pour le raccordement d'au moins un connecteur de l'unité fiche, de sorte que par le raccordement du connecteur à la pièce de raccordement il est possible d'établir une liaison d'écoulement entre le dispositif médical et le dispositif pour la mise à disposition de liquides médicaux pour l'introduction de liquide frais ou l'évacuation de liquide usagé,
**caractérisé en ce que**
l'unité douille (B) présente un mécanisme de raccordement pour l'établissement d'une liaison entre la au moins une pièce de raccordement (27, 28) et le au moins un connecteur, où le mécanisme de raccordement présente une unité d'entraînement qui présente au moins un corps d'entraînement mobile (150) qui décrit un mouvement de translation ou de rotation,
au moins un mécanisme de saisie de position pour la saisie de la position linéaire ou de la position angulaire du corps d'entraînement (150) est prévu, et
le mécanisme de saisie de position présente :
au moins deux éléments d'aimants (100, 110) produisant un champ magnétique disposés à distance l'un de l'autre et au moins deux éléments capteurs (120, 130) saisissant le champ magnétique des éléments d'aimants, où les au moins deux éléments d'aimants sont disposés sur le corps d'entraînement mobile et les au moins deux éléments capteurs sont disposés de manière fixe par rapport au corps d'entraînement mobile ou les au moins deux éléments d'aimants sont disposés de manière fixe par rapport au corps d'entraînement mobile et les au moins deux éléments capteurs sont disposés sur le corps d'entraînement mobile, et
une unité d'évaluation (180), qui est configurée de telle façon que la position linéaire ou la position angulaire du corps d'entraînement (150) est déterminée sur la base du champ magnétique produit par les au moins deux éléments d'aimants (100, 110) et saisi par les au moins deux éléments capteurs (120, 130), où
les au moins deux éléments d'aimants (100, 110) du mécanisme de saisie de position sont au moins un premier élément d'aimant (100) et un second élément d'aimant (110), qui sont disposés avec une polarité opposée l'un par rapport à l'autre à distance l'un de l'autre, et les au moins deux éléments capteurs (120, 130) du mécanisme de saisie de position sont au moins un premier élément capteur (120) et un second élément capteur (130), qui sont disposés à distance l'un de l'autre, où l'unité d'évaluation (180) est configurée de telle façon que sur la base du champ magnétique saisi par le premier élément capteur (120) deux positions linéaires ou positions angulaires possibles du corps d'entraînement (150) sont déterminées et sur la base du champ magnétique saisi par le second élément capteur (130) on détermine si la position linéaire ou la position angulaire déterminée par le premier élément capteur est la première ou la seconde position ou position angulaire des deux positions ou positions angulaires possibles.

2. Dispositif médical selon la revendication 1, **caractérisé en ce que** l'unité d'évaluation (180) est configurée de telle manière que sur la base du sens du champ magnétique saisi par le second élément capteur (130) on détermine si la position linéaire ou la position angulaire saisie par le premier élément capteur (120) est la première ou seconde position ou position angulaire des deux positions ou positions angulaires possibles.

3. Dispositif médical avec une unité douille (B) pour le raccordement d'une unité fiche d'un dispositif pour la mise à disposition de liquides médicaux,
où l'unité douille (B) présente au moins une pièce de raccordement (27, 28) pour le raccordement d'au moins un connecteur de l'unité fiche, de sorte que par le raccordement du connecteur à la pièce de raccordement il est possible d'établir une liaison d'écoulement entre le dispositif médical et le dispositif pour la mise à disposition de liquides médicaux pour l'introduction de liquide frais ou l'évacuation de liquide usagé,
**caractérisé en ce que**
l'unité douille (B) présente un mécanisme de raccordement pour l'établissement d'une liaison entre la au moins une pièce de raccordement (27, 28) et le au moins un connecteur, où le mécanisme de raccordement présente une unité d'entraînement qui présente au moins un corps d'entraînement mobile (150) qui décrit un mouvement de translation,
au moins un mécanisme de saisie de position pour la saisie de la position linéaire du corps d'entraînement (150) est prévu, où
le mécanisme de saisie de position présente :
au moins deux éléments d'aimants (100, 110) produisant un champ magnétique disposés à distance l'un de l'autre et au moins un élément capteur (120) saisissant le champ magnétique des éléments d'aimants, où les au moins deux éléments d'aimants sont disposés sur le corps d'entraînement mobile et le au moins un élément capteur est disposé de manière fixe par rapport au corps d'entraînement mobile ou les au moins deux éléments d'aimants sont disposés de manière fixe par rapport au corps d'entraînement mobile et le au moins un élément capteur est disposé sur le corps d'entraînement mobile, et
une unité d'évaluation (180), qui est configurée de telle façon que la position linéaire du corps d'entraînement (150) est déterminée sur la base du champ magnétique produit par les au moins deux éléments d'aimants (100, 110) et saisi par le au moins un élément capteur (120), où
le mouvement de translation du corps d'entraînement (150) est limité par un ou plusieurs éléments de butée (140, 145).

4. Dispositif médical avec une unité douille (B) pour le raccordement d'une unité fiche d'un dispositif pour la mise à disposition de liquides médicaux,
où l'unité douille (B) présente au moins une pièce de raccordement (27, 28) pour le raccordement d'au moins un connecteur de l'unité fiche, de sorte que par le raccordement du connecteur à la pièce de raccordement il est possible d'établir une liaison d'écoulement entre le dispositif médical et le dispositif pour la mise à disposition de liquides médicaux pour l'introduction de liquide frais ou l'évacuation de liquide usagé,
**caractérisé en ce que**
l'unité douille (B) présente un mécanisme de raccordement pour l'établissement d'une liaison entre la au moins une pièce de raccordement (27, 28) et le au moins un connecteur, où le mécanisme de raccordement présente une unité d'entraînement qui présente au moins un corps d'entraînement mobile (150) qui décrit un mouvement de rotation,
au moins un mécanisme de saisie de position pour la saisie de la position angulaire du corps d'entraînement (150) est prévu, où
le mécanisme de saisie de position présente :
au moins deux éléments d'aimants (100, 110) produisant un champ magnétique disposés à distance l'un de l'autre et au moins un élément capteur (120) saisissant le champ magnétique des éléments d'aimants, où les au moins deux éléments d'aimants sont disposés sur le corps d'entraînement mobile et le au moins un élément capteur est disposé de manière fixe par rapport au corps d'entraînement mobile ou les au moins deux éléments d'aimants sont disposés de manière fixe par rapport au corps d'entraînement mobile et le au moins un élément capteur est disposé sur le corps d'entraînement mobile, et
une unité d'évaluation (180), qui est configurée de telle façon que la position angulaire du corps d'entraînement (150) est déterminée sur la base du champ magnétique produit par les au moins deux éléments d'aimants (100, 110) et saisi par le au moins un élément capteur (120), où
le mouvement de rotation du corps d'entraînement (150) est limité par un ou plusieurs éléments de butée (140, 145).

5. Dispositif médical avec une unité douille (B) pour le raccordement d'une unité fiche d'un dispositif pour la mise à disposition de liquides médicaux,
où l'unité douille (B) présente au moins une pièce de raccordement (27, 28) pour le raccordement d'au moins un connecteur de l'unité fiche, de sorte que par le raccordement du connecteur à la pièce de raccordement il est possible d'établir une liaison d'écoulement entre le dispositif médical et le dispositif pour la mise à disposition de liquides médicaux pour l'introduction de liquide frais ou l'évacuation de liquide usagé,
**caractérisé en ce que**
l'unité douille (B) présente un mécanisme de raccordement pour l'établissement d'une liaison entre la au moins une pièce de raccordement (27, 28) et le au moins un connecteur, où le mécanisme de raccordement présente une unité d'entraînement qui présente au moins un corps d'entraînement mobile (150) qui décrit un mouvement de translation ou de rotation,
au moins un mécanisme de saisie de position pour la saisie de la position linéaire ou de la position angulaire du corps d'entraînement (150) est prévu, où
le mécanisme de saisie de position présente :
au moins deux éléments d'aimants (100, 110) produisant un champ magnétique disposés à distance l'un de l'autre et au moins un élément capteur (120) saisissant le champ magnétique des éléments d'aimants, où les au moins deux éléments d'aimants sont disposés sur le corps d'entraînement mobile et le au moins un élément capteur est disposé de manière fixe par rapport au corps d'entraînement mobile ou les au moins deux éléments d'aimants sont disposés de manière fixe par rapport au corps d'entraînement mobile et le au moins un élément capteur est disposé sur le corps d'entraînement mobile, et
une unité d'évaluation (180), qui est configurée de telle façon que la position linéaire du corps d'entraînement (150) est déterminée sur la base du champ magnétique produit par les au moins deux éléments d'aimants (100, 110) et saisi par le au moins un élément capteur (120), où
les au moins deux éléments d'aimants (100, 110) du mécanisme de saisie de position sont au moins un premier élément d'aimant (100) et un second élément d'aimant (110), qui sont disposés avec une polarité opposée l'un par rapport à l'autre à distance l'un de l'autre, où l'unité d'évaluation (180) est configurée de telle façon que sur la base du champ magnétique saisi par le au moins un élément capteur (120) deux positions linéaires et/ou positions angulaires possibles du corps d'entraînement sont déterminées, et **en ce que** l'unité d'évaluation (180) est configurée de telle façon que le gradient de la variation du champ magnétique saisi par l'élément capteur (120) lors d'une variation de la position linéaire ou de la position angulaire du corps d'entraînement est déterminé, et sur la base du sens du gradient de la variation, on détermine si la position linéaire ou la position angulaire déterminée avec le premier élément capteur est la première ou seconde position ou position angulaire des deux positions ou positions angulaires possibles.

6. Dispositif médical selon l'une des revendications 1 à 5, **caractérisé en ce que** l'unité d'entraînement du mécanisme de raccordement présente un corps d'entraînement (150) qui présente des moyens pour la liaison amovible de l'unité fiche avec l'unité douille.

7. Dispositif médical selon la revendication 6, **caractérisé en ce que** les moyens pour la liaison amovible de l'unité fiche avec l'unité douille présentent une pièce de réception (43) pour enficher un embout de l'unité fiche.

8. Dispositif médical selon l'une des revendications 1 à 7, **caractérisé en ce que** l'unité d'évaluation (180) produit un premier signal de commande quand le mécanisme de saisie de position détecte une première position avancée du corps d'entraînement (150), dans laquelle le au moins un connecteur et la au moins une pièce de raccordement sont séparés, et produit un second signal de commande quand le mécanisme de saisie de position détecte une seconde position reculée du corps d'entraînement (150), dans laquelle le au moins un connecteur est raccordé à la au moins une pièce de raccordement.

9. Dispositif médical selon l'une des revendications 1 à 5, **caractérisé en ce que** le corps d'entraînement (150) de l'unité d'entraînement du mécanisme de raccordement peut tourner autour d'un axe de rotation (25).

10. Dispositif médical selon la revendication 9, **caractérisé en ce que** la au moins une pièce de raccordement (26, 27) de l'unité douille (B) est entourée de manière concentrique par une partie de raccordement (23, 24) avec formation d'une chambre de rinçage (28, 49) et l'unité douille (B) présente un obturateur (50) avec au moins une pièce de fermeture (51, 52) pour la fermeture de la au moins une chambre de rinçage, où
l'obturateur (50) est prévu sur le corps d'entraînement pouvant tourner et la au moins une pièce de fermeture (51, 52) est disposée sur l'obturateur (50) à distance de l'axe de rotation, et
dans une première position de rotation la au moins une pièce de fermeture (51, 52) et la au moins une partie de raccordement (23, 24) sont situées sur un axe commun, de sorte que par un mouvement relatif de l'unité fiche et de l'unité douille (B) il est possible d'établir une liaison entre la pièce de fermeture et la partie de raccordement pour la fermeture de la chambre de rinçage, et
dans une seconde position de rotation la au moins une pièce de fermeture (51, 52) et la au moins une pièce de raccordement (23, 24) sont disposées décalées l'une par rapport à l'autre, de sorte que lors de l'enfichage de l'unité fiche dans l'unité douille (B) il est possible d'établir une liaison entre le au moins un connecteur (29, 30) de l'unité fiche (A) et la au moins une pièce de raccordement (26, 27) de l'unité douille (B).

11. Dispositif médical selon la revendication 10, **caractérisé en ce que** l'unité d'évaluation (180) produit un premier signal de commande quand le mécanisme de saisie de position détecte la première position de rotation et produit un second signal de commande quand le mécanisme de saisie de position détecte la seconde position de rotation.

12. Dispositif médical selon l'une des revendications 1 à 11, **caractérisé en ce que** l'élément d'aimant (100, 110) est un aimant permanent.

13. Dispositif médical selon l'une des revendications 1 à 12, **caractérisé en ce que** l'élément capteur (120) est un capteur à effet Hall.

14. Dispositif médical selon l'une des revendications 1 à 13, **caractérisé en ce que** le dispositif médical est un dispositif de traitement du sang (2), en particulier un dispositif de dialyse extracorporel ou un dispositif pour la dialyse péritonéale.
